(19) **Europäisches Patentamt**
**European Patent Office**
**Office européen des brevets**

(11) **EP 4 270 305 A1**

(12) **EUROPEAN PATENT APPLICATION**
published in accordance with Art. 153(4) EPC

(43) Date of publication:
**01.11.2023 Bulletin 2023/44**

(21) Application number: **21910426.2**

(22) Date of filing: **13.12.2021**

(51) International Patent Classification (IPC):
**G06T 7/00** (2017.01)      **A61B 6/00** (2006.01)
**G06N 20/00** (2019.01)

(52) Cooperative Patent Classification (CPC):
**A61B 6/00; G06N 20/00; G06T 7/00**

(86) International application number:
**PCT/JP2021/045790**

(87) International publication number:
**WO 2022/138277 (30.06.2022 Gazette 2022/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.12.2020 JP 2020214722**

(71) Applicant: **FUJIFILM Corporation
Tokyo 106-8620 (JP)**

(72) Inventor: **HIASA, Yuta
Tokyo 106-8620 (JP)**

(74) Representative: **Klunker IP
Patentanwälte PartG mbB
Destouchesstraße 68
80796 München (DE)**

(54) **LEARNING DEVICE, METHOD, AND PROGRAM, AND MEDICAL IMAGE PROCESSING DEVICE**

(57)      Provided are a learning device, a learning method, a learning program, and a medical use image processing device capable of reducing an annotation cost for preparing a data set for learning and creating a learning model with high reliability of detection of a disease. A first processor of a learning device reads out a first medical use image (a1) having a disease label (x) from a data set (B) stored in a memory (14) and inputs the read out first medical use image (a1) to a first learning model (16). The first medical use image (a1) is normalized based on a lung field region estimated by the first learning model (16), and a second learning model (18) that has not been trained and detects a disease is trained by using the normalized first medical use image (A1) and the disease label (x). In a case in which the second learning model (18) is trained, a value (C1) of the uncertainty of the first medical use image (a1) is calculated based on the uncertainty (c1) simultaneously estimated by the first learning model (16), and the first medical use image (a1) having a large value of uncertainty (C1) is excluded from learning data.

FIG. 2

EP 4 270 305 A1

**Description**

**BACKGROUND OF THE INVENTION**

1. Field of the Invention

The present invention relates to a learning device, a learning method, a learning program, and a medical use image processing device, and more particularly relates to a technique of generating a learning model for detecting a disease or the like from a medical use image.

2. Description of the Related Art

Baltruschat, et al., "When does bone suppression and lung field segmentation improve chest x-ray disease classification?", ISBI, 2019. describes that bone weakening processing and cropping of a lung field region are performed as preprocessing and how much the processing contributes to the detection accuracy of a lung disease by deep learning, and describes that cropping of the lung field region improves area under the curve (AUC) from 0.891 $\pm$ 0.013 to 0.905 $\pm$ 0.012, that is, the performance of the deep learning can be improved.

In addition, in Ghesu, et al., "Quantifying and leveraging classification uncertainty for chest radiograph assessment.", MICCAI, 2019., there is a description regarding the estimation and application of uncertainty in the detection of the lung disease. Interpretation of a chest X-ray image has subjective ambiguity about the definition of the disease and is not easy to perform annotation. Therefore, it is assumed that noise is added to labeled data used for the deep learning. In Ghesu, et al., "Quantifying and leveraging classification uncertainty for chest radiograph assessment.", MICCAI, 2019., the uncertainty of the network and a class probability are output to follow beta distribution and a weight parameter is optimized by using an error function that minimizes the Bayes risk.

On the other hand, JP2020-91862A discloses a method of processing a medical use image by a Bayesian deep learning network, determining a first image feature of interest located in a first sub-region of the medical use image and a value of associated uncertainty, processing the medical use image by a hostile generation network in the same manner, determining a second image feature of interest in the first sub-region of the medical use image and a value of associated uncertainty, and classifying the first sub-region of the medical use image based on the first and second image features and the values of associated uncertainty.

**SUMMARY OF THE INVENTION**

As described in Baltruschat, et al., "When does bone suppression and lung field segmentation improve chest x-ray disease classification?", ISBI, 2019., in a case in which an organ region (lung field region) is cropped, it is necessary to manually create a correct answer region (correct answer data) indicating the lung field region for all medical use images for learning to be used for deep learning, and there is a problem that an annotation cost is increased in a case of large-scale data.

In Ghesu, et al., "Quantifying and leveraging classification uncertainty for chest radiograph assessment.", MICCAI, 2019., only the uncertainty of the class-classification is targeted, and the uncertainty of the organ extraction is not taken into consideration.

In addition, in JP2020-91862A, the values of uncertainty of the medical use images acquired by different networks (Bayesian deep learning network and hostile generation network) and the like are used to classify the first sub-region of the medical use image, and are not used for the learning of the network.

The present invention has been made in view of such circumstances, and is to provide a learning device, a learning method, a learning program, and a medical use image processing device capable of reducing an annotation cost for preparing a data set for learning and capable of creating a learning model with high reliability of detection of the disease.

In order to achieve the above object, a first aspect of the present invention relates to a learning device comprising a first processor, a memory that stores a data set for learning consisting of a plurality of first medical use images each having a disease label, a first learning model that has been trained and estimates extraction of an organ region and uncertainty for the extraction of the organ region from the first medical use image in a case in which the first medical use image is input, and a second learning model that has not been trained and detects a disease from any second medical use image, in which the first processor performs processing of reading out the first medical use image from the memory and inputting the read out first medical use image to the first learning model, processing of normalizing the first medical use image to be input to the second learning model based on the organ region estimated (the estimated organ region) by the first learning model or setting information indicating the organ region with respect to the first medical use image, processing of calculating a value of uncertainty of the first medical use image input to the first learning model based on the uncertainty estimated by the first learning model, and processing of training the second learning model by using the normalized first medical use image, or the first medical use image and the information indicating the organ region as input data, and the disease label of the first medical use image as a correct answer label, in which the second learning model is trained by reflecting the calculated value of uncertainty of the first medical use image.

According to the first aspect of the present invention, in a case in which large-scale data (100,000 or more medical use images) are prepared as the data set for learning, labeling of an organ region is unnecessary, and the annotation cost can be reduced. In addition, the first medical use image is normalized based on the organ region estimated by the first learning model, or the information indicating the organ region is set with respect to the first medical use image, and further the second learning model is trained by reflecting the value of uncertainty of the first medical use image in a case in which the second learning model is trained, so that the second learning model with high reliability of the detection of the disease can be created.

In the learning device according to a second aspect of the present invention, it is preferable that, in the processing of training the second learning model, it is determined whether or not the calculated value of uncertainty of the first medical use image is smaller than a threshold value, and the first medical use image for which it is determined that the value of uncertainty is smaller than the threshold value among the first medical use images constituting the data set is used. The first medical use image for which it is determined that the value of uncertainty is smaller than the threshold value among the first medical use images constituting the data set is used (first medical use image, which has the value of uncertainty equal to or larger than the threshold value and is inappropriate for training the second learning model, is excluded), so that the second learning model with high reliability of the detection of the disease can be created.

In the learning device according to a third aspect of the present invention, it is preferable that, in the processing of training the second learning model, an error between an estimation result of the second learning model and the disease label is weighted according to the calculated value of uncertainty of the first medical use image, and the second learning model is trained based on the weighted error. For example, in a case in which the value of uncertainty of the first medical use image is high (in a case in which the first medical use image is inappropriate for training the second learning model), the weighting for the error between the estimation result of the second learning model and the disease label is reduced, and an influence on training the second learning model is reduced. As a result, the second learning model is trained by reflecting the value of uncertainty for each first medical use image, so that the second learning model with high reliability of the detection of the disease can be created.

In the learning device according to a fourth aspect of the present invention, it is preferable that the processing of normalizing the first medical use image includes processing of cutting out the estimated organ region from the first medical use image.

In the learning device according to a fifth aspect of the present invention, it is preferable that, in the processing of normalizing the first medical use image, a pixel value of the first medical use image is normalized by a statistic of a pixel value belonging to the organ region estimated from the first medical use image.

In the learning device according to a sixth aspect of the present invention, it is preferable that the first learning model is a learning model consisting of a Bayesian neural network.

In the learning device according to a seventh aspect of the present invention, it is preferable that the second learning model is a learning model consisting of densely connected convolutional networks (DenseNet).

In the learning device according to an eighth aspect of the present invention, it is preferable that the first medical use image and the second medical use image are chest X-ray images, respectively.

In the learning device according to a ninth aspect of the present invention, it is preferable that the disease is a lung nodule.

A tenth aspect of the present invention relates to a medical use image processing device comprising the learning device according to any one of the first to ninth aspects, and a second processor, in which the second processor performs processing of inputting the second medical use image to the first learning model, and normalizing the second medical use image based on the organ region estimated by the first learning model or setting the information indicating the organ region with respect to the second medical use image, and processing of inputting the normalized second medical use image, or the second medical use image and the information indicating the organ region to the second learning model that has been trained, and detecting the disease from the second medical use image based on an estimation result of the second learning model.

According to the tenth aspect of the present invention, the first learning model and the second learning model that has been trained by the learning device according to any one of the first to ninth aspects is used, and the second medical use image that is a target for the detection of the disease is input to the first learning model, so that the second medical use image is normalized based on the organ region estimated by the first learning model, or the information indicating the organ region is set with respect to the second medical use image. Then, the normalized second medical use image, or the second medical use image and the information indicating the organ region can be input to the second learning model that has been trained, and the disease can be detected from the second medical use image based on the estimation result of the second learning model. The normalized second medical use image, or the second medical use image and the information indicating the organ region are used as the input data of the second learning model, so that the detection accuracy of the disease or the like can be improved.

In the medical use image processing device according to an eleventh aspect of the present invention, it is preferable that the second processor performs processing of inputting the second medical use image to the first learning model, processing of calculating a value of uncertainty of the second medical use image input to the first learning model based on the uncertainty estimated by the first learning model, and determining whether or not the value of uncertainty is smaller than a threshold value, and processing of notifying a user of a warning in a case in which it is determined that the value of uncertainty of the second medical use image is larger than the threshold value. The reason is that, in a case in which the second medical use image having a large value of uncertainty is input to the second learning model, there is a possibility that the detection result with high reliability is not obtained, and the warning against the possibility is given. It should be noted that the second medical use image having a large value of uncertainty may not be input to the second learning model, and in this case, the notification of the warning also means that the detection of the disease by the second learning model is not performed. In addition, the second medical use image having a large value of uncertainty may be input to the second learning model, and in this case, the notification of the warning means that the reliability of the detection result of the disease by the second learning model is low.

In the medical use image processing device according to a twelfth aspect of the present invention, it is preferable that the value of uncertainty of the second medical use image is calculated as a statistic converted per unit area of the organ region based on the uncertainty of the second medical use image input to the first learning model, and in the processing of notifying the user of the warning, an icon indicating the statistic or the warning is displayed.

In the medical use image processing device according to a thirteenth aspect of the present invention, it is preferable that, in the processing of notifying the user of the warning, uncertainty of the organ region of the second medical use image as well as the second medical use image is displayed as a heat map.

In the medical use image processing device according to a fourteenth aspect of the present invention, it is preferable that, in the processing of notifying the user of the warning, a contour or a circumscribing figure of a region obtained by binarizing uncertainty of the organ region of the second medical use image as well as the second medical use image is displayed.

A fifteenth aspect of the present invention relates to a learning method in which a first processor trains a second learning model that has not been trained and detects a disease from any second medical use image, by using a data set for learning that is stored in a memory and consists of a plurality of first medical use images each having a disease label, and a first learning model that has been trained and estimates extraction of an organ region and uncertainty for the extraction of the organ region from the first medical use image in a case in which the first medical use image is input, the learning method comprising a step of reading out the first medical use image from the memory and inputting the read out first medical use image to the first learning model, a step of normalizing the first medical use image to be input to the second learning model based on the organ region estimated by the first learning model or setting information indicating the organ region with respect to the first medical use image, a step of calculating a value of uncertainty of the first medical use image input to the first learning model based on the uncertainty estimated by the first learning model, and a step of training the second learning model by using the normalized first medical use image, or the first medical use image and the information indicating the organ region as input data, and the disease label of the first medical use image as a correct answer label, in which the second learning model is trained by reflecting the calculated value of uncertainty of the first medical use image.

In the learning method according to a sixteenth aspect of the present invention, it is preferable that, in the step of training the second learning model, it is determined whether or not the calculated value of uncertainty of the first medical use image is smaller than a threshold value, and the first medical use image for which it is determined that the value of uncertainty is smaller than the threshold value among the first medical use images constituting the data set is used.

In the learning method according to a seventeenth aspect of the present invention, it is preferable that, in the step of training the second learning model, an error between an estimation result of the second learning model and the disease label is weighted according to the calculated value of uncertainty of the first medical use image, and the second learning model is trained based on the weighted error.

An eighteenth aspect of the present invention relates to a learning program causing the first processor to execute processing of each step in the learning method according to any one of the fifteenth to seventeenth aspects.

According to the present invention, it is possible to reduce the annotation cost for preparing the data set for learning, and it is possible to create the learning model with high reliability of the detection of the disease as the learning model that detects the disease from the medical use image.

**BRIEF DESCRIPTION OF THE DRAWINGS**

Fig. 1 is a block diagram showing an embodiment of a hardware configuration of a learning device and a medical use image processing device according to an embodiment of the present invention.

Fig. 2 is a functional block diagram showing a first embodiment of the learning device according to the embodiment of the present invention.

Fig. 3 is a diagram including a first learning model and being used for describing a function of the first learning model.

Fig. 4 is a diagram showing an example of a second learning model.

Fig. 5 is a functional block diagram showing a second embodiment of the learning device according to the embodiment of the present invention.

Fig. 6 is a flowchart showing a first embodiment of a learning method according to the embodiment of the present invention.

Fig. 7 is a flowchart showing a second embodiment of the learning method according to the embodiment of the present invention.

Fig. 8 is a functional block diagram showing an embodiment of a medical use image processing device according to the embodiment of the present invention.

Fig. 9 is a diagram showing a first display example on a display screen of a display unit.

Fig. 10 is a diagram showing a second display example on the display screen of the display unit.

Fig. 11 is a chart showing an example of a patient list.

## DESCRIPTION OF THE PREFERRED EMBODIMENTS

Hereinafter, preferred embodiments of a learning device, a learning method, and a learning program, and a medical use image processing device according to the embodiment of the present invention will be described with reference to the accompanying drawings.

[Hardware Configuration of Learning Device and Medical Use Image Processing Device]

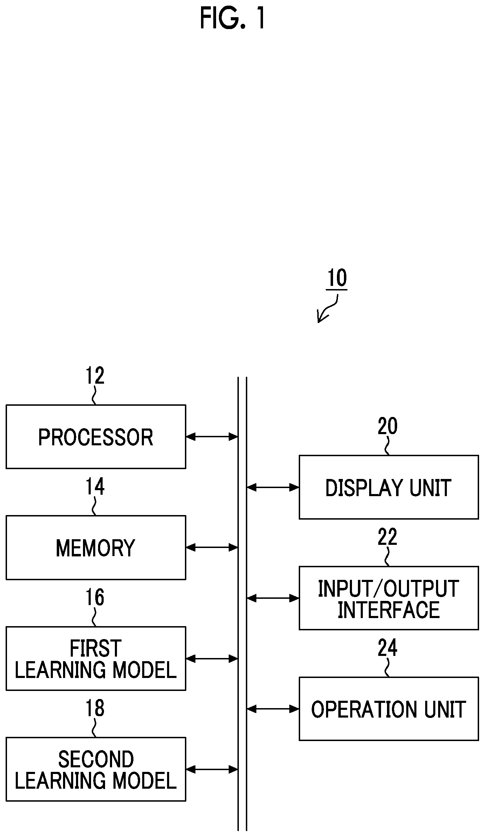

Fig. 1 is a block diagram showing an embodiment of a hardware configuration of the learning device and the medical use image processing device according to the embodiment of the present invention. It should be noted that the medical use image processing device according to this example includes a function as the learning device.

A medical use image processing device 10 shown in Fig. 1 can be configured by a personal computer, a workstation, or the like, and comprises a processor 12, a memory 14, a first learning model 16, a second learning model 18, a display unit 20, an input/output interface 22, an operation unit 24, and the like.

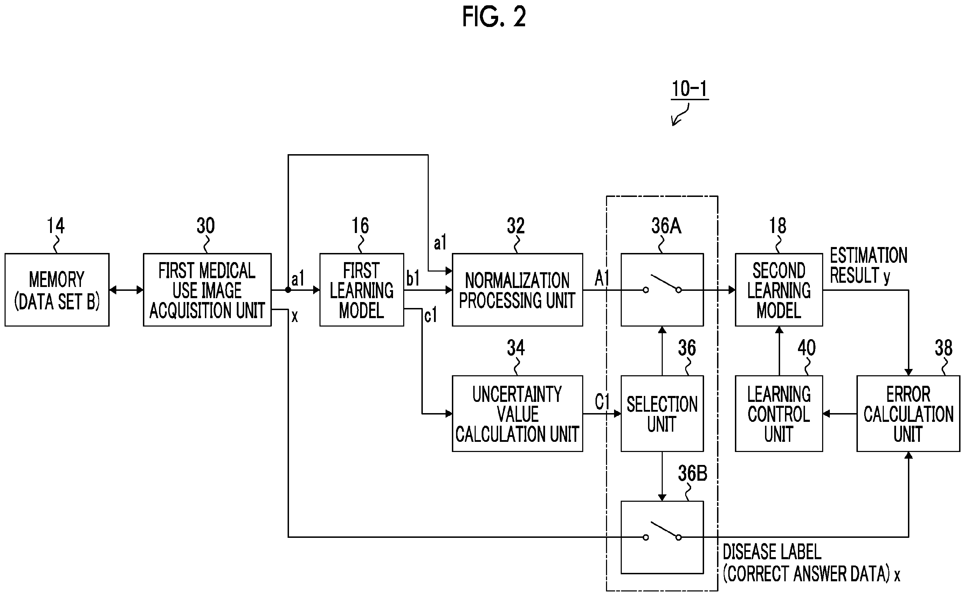

The processor 12 is configured by a central processing unit (CPU) or the like, and controls the respective units of the medical use image processing device 10 in an integrated manner to function as a first medical use image acquisition unit 30, a normalization processing unit 32, a uncertainty value calculation unit 34, a selection unit 36, and a processing unit (error calculation unit 38 and learning control unit 40) that trains a second learning model 18 shown in Fig. 2, for example.

The memory 14 includes a flash memory, a read-only memory (ROM), a random access memory (RAM), a hard disk apparatus, and the like. The flash memory, the ROM, and the hard disk apparatus are non-volatile memories that store various programs including a learning program causing the processor 12 to execute the operation system and the processing of each step in the learning method according to the embodiment of the present invention. In addition, the hard disk apparatus can function as a large-capacity memory that stores a data set for learning described later.

The RAM functions as a work region for processing by the processor 12, and also transitorily stores the learning program or the like stored in the non-volatile memory. It should be noted that a part (RAM) of the memory 14 may be built in the processor 12.

The first learning model 16 is a learning model that simultaneously estimates the extraction of an organ region and the uncertainty for the extraction of the organ region from the medical use image (first medical use image for learning with respect to the second learning model), is preferably a learning model consisting of a Bayesian neural network, and more preferably a Bayesian U-Net.

The first learning model 16 in the learning device may be a trained learning model or a learning model that has not been trained. In a case of the first learning model 16 that has not been trained, the first learning model 16 need only be trained by the processor 12 or the like by using a data set for learning (data set A) corresponding to the first learning model 16.

The data set A in this case is a relatively small-scale data set consisting of a plurality of pairs of a third medical use image and an organ label (correct answer label). As the third medical use image, a medical use image suitable for learning, which has less noise or blurriness, is selected, and the organ label can be created as a mask image corresponding to the organ region by displaying the third medical use image on a display and filling a desired organ region on a pixel unit by a user (for example, doctor). In addition, the organ label may be labeled to be distinguishable for each organ.

Since the learning method of the first learning model 16 using this type of the data set A can be performed by a known method, the detailed description thereof will be omitted. In addition, in the Bayesian neural network, since desired learning can be performed even with a small-scale data set A, an annotation cost for preparing the data set A can be reduced.

The second learning model 18 is a learning model that detects a disease from the medical use image (any second medical use image that is a diagnosis target), and is preferably a learning model consisting of densely connected convolutional networks (DenseNet).

The second learning model 18 in the learning device has not been trained, and the learning device according to the embodiment of the present invention trains the second learning model 18 through machine learning.

A data set for learning (data set B) corresponding to the second learning model 18 is a plurality of medical use images (first medical use images) each having a disease label, and is large-scale data consisting of, for example, 100,000 or more first medical use images. The large-scale data set B is a first medical use image group with the disease label (information indicating the presence or absence of the disease or a disease name), such a first medical use image group can be acquired by accumulating the medical use images during image diagnosis, and the annotation cost is low.

Details of the learning device and the learning method according to the embodiment of the present invention that train the second learning model 18 by using the data set B will be described later.

On the other hand, the first learning model 16 and the second learning model 18 in the medical use image processing device 10 in a case in which image diagnosis support is performed are trained models, and in particular, the second learning model 18 is the learning model that has been trained by the learning device provided in the medical use image processing device 10.

It should be noted that the processor 12 according to this example includes a processor (first processor) for the learning device that functions in a case in which the second learning model 18 is trained, and a processor (second processor) for the medical use image processing device that detects the disease from the medical use image that is the diagnosis target by using the first learning model 16 and the second learning model 18, which have been trained, respectively, but the first processor and the second processor may be physically different from each other.

Also, the first learning model 16 and the second learning model 18 may be realized by the processor 12 by a program corresponding to each learning model.

Further, the medical use images (first medical use image, second medical use image, and third medical use image) according to this example are, for example, chest X-ray images, the organ region is a lung field region, and a lung disease to be detected by the second learning model 18 that has been trained is a lung nodule (oval shadow).

The display unit 20 is a display that displays the second medical use image, a detection result, a warning, and the like in a case in which the disease is detected from the second medical use image that is the diagnosis target. In a case in which the second medical use image that is the diagnosis target is observed and the image diagnosis is performed, the user (doctor) can make a diagnosis with reference to the detection result and the like displayed on the display unit 20. It should be noted that the display unit 20 can be used as a part of a user interface in a case in which various instructions of the user are received.

The input/output interface 22 includes a connection unit that can be connected to an external apparatus, a communication unit that can be connected to a network, and the like. As the connection unit that can be connected to the external apparatus, a universal serial bus (USB), a high-definition multimedia interface (HDMI) (HDMI is a registered trademark), or the like can be applied. The processor 12 can acquire various programs including the learning program, the data set B, and the like, which are stored in the memory 14, in addition to the second medical use image that is the diagnosis target, via the input/output interface 22. In addition, it is possible to use an external display apparatus connected to the input/output interface 22 instead of the display unit 20.

The operation unit 24 includes a pointing device, such as a keyboard and a mouse, and a keyboard, and functions as a user interface that receives various designations by the diagnostician.

[Learning Device]

<First Embodiment of Learning Device>

Fig. 2 is a functional block diagram showing a first embodiment of the learning device according to the embodiment of the present invention.

A learning device 10-1 according to the first embodiment shown in Fig. 2 is configured by the processor 12 (first processor), the memory 14, the first learning model 16, and the second learning model 18 provided in the medical use image processing device 10 of the structure having the hardware configuration shown in Fig. 1, and the processor 12 functions as the first medical use image acquisition unit 30, the normalization processing unit 32, the uncertainty value calculation unit 34, the selection unit 36, and a processing unit (error calculation unit 38 and learning control unit 40) that trains a second learning model 18.

The first medical use image acquisition unit 30 reads out the first medical use image with the disease label from the memory 14 that stores the data set B for learning, outputs a read out first medical use image a1 to the first learning model 16 and the normalization processing unit 32, and outputs a disease label x added to the first medical use image a1 to the error calculation unit 38 via the switch 36B as the correct answer label (correct answer data).

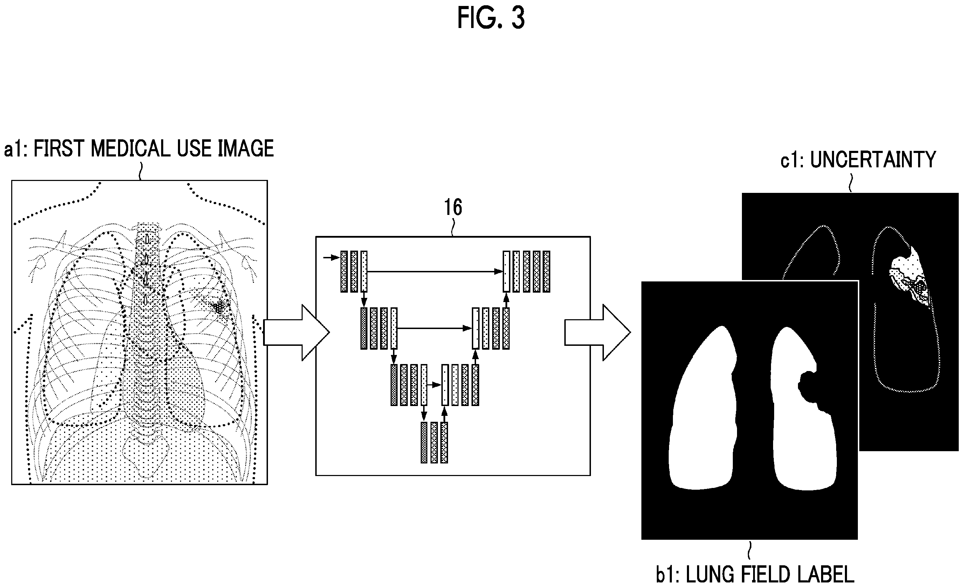

Fig. 3 is a diagram including the first learning model and being used for describing the function of the first learning model.

The first learning model 16 shown in Fig. 3 is a Bayesian U-Net, which is a trained neural network that extracts the organ region from the medical use image (first medical use image).

The first learning model 16 extracts the organ region (lung field region in this example) from the first medical use image a1 in a case in which the first medical use image (chest X-ray image) a1 as shown in Fig. 3 is input, and outputs a lung field label (binary mask image indicating whether or not it is the lung field region) b 1 indicating the lung field region and uncertainty c1 for the extraction of the lung field region.

The uncertainty c1 is a predictive variance for each pixel estimated by the first learning model 16. The uncertainty is low in a case in which the variance is small, and the uncertainty is high in a case in which the variance is large. The definition is made by the following expression.

$$Var(y^* = c|\mathbf{x}^*, \mathbf{X}, \mathbf{Y})$$
$$\approx \frac{1}{T} \sum_{t=1}^{T} \mathrm{Softmax}(\mathbf{f}(\mathbf{x}^*, \hat{\mathbf{W}}))^T \mathrm{Softmax}(\mathbf{f}(\mathbf{x}^*, \hat{\mathbf{W}}))$$
$$-p(y^*|\mathbf{x}^*, \mathbf{X}, \mathbf{Y})^T p(y^*|\mathbf{x}^*, \mathbf{X}, \mathbf{Y}).$$

(See "Gal, Yarin, and Zoubin Ghahramani. "Dropout as a Bayesian approximation: Representing model uncertainty in deep learning.", ICML, 2016.")

Since the Monte Carlo dropout (MC dropout) is used, a parameter W of the network follows the Bernoulli distribution and behaves probabilistically. In addition, $\mathrm{Softmax}(f(x^*, W))$ is the output of the network, and T is the number of repetitions of MCMC. That is, T images of the same image are input to the network, and the variance of the outputs is calculated.

In addition, the Bayesian U-Net, which is the first learning model 16, is an extension of the U-Net such that the uncertainty can be estimated by using the MC dropout.

(See "Hiasa, Yuta, et al. "Automated muscle segmentation from clinical CT using Bayesian U-net for personalized musculoskeletal Modeling." IEEE TMI, 2019.").

Also, the uncertainty can be broadly divided into epistemic uncertainty and aleatory uncertainty. The epistemic uncertainty is the uncertainty for a sample outside the distribution of the learning data, and the aleatory uncertainty is the uncertainty for the noise of the data.

It should be noted that the uncertainty c1 shown in Fig. 3 is a heat map showing the uncertainty of the extracted lung field region.

The first learning model 16 outputs the lung field label b 1 estimated from the input first medical use image a1 to the normalization processing unit 32, and outputs the simultaneously estimated uncertainty c1 to the uncertainty value calculation unit 34.

The first medical use image a1 is added to another input of the normalization processing unit 32, and the normalization processing unit 32 normalizes the first medical use image a1 based on the lung field label b1 (lung field region indicated by the lung field label b1). The processing of normalizing the first medical use image a1 by the normalization processing unit 32 includes processing of cutting out the estimated lung field region (the lung field region estimated) from the first medical use image a1. For example, an image in a rectangular frame (bounding box) circumscribed to the lung field region may be cut out from the first medical use image a1, or may be cut out with a margin added to a bounding box. In addition, an image size and/or an aspect ratio of the lung field region cut out by the bounding box may be resized to be regular. Further, only the lung field region may be cut out.

In addition, it is preferable that the normalization processing unit 32 normalizes a pixel value of the first medical use image a1 with a statistic of a pixel value belonging to the lung field region estimated from the first medical use image a1. Examples of the statistic of the pixel value belonging to the lung field region include an average value, a maximum value, and a minimum value of the pixel values belonging to the lung field region, and it is preferable that the density adjustment of the image belonging to the lung field region is performed with these statistics. For example, the average value of the pixel values belonging to the lung field region is normalized to be a predetermined value (128 of 256 gradations), and a dynamic range of the image of the lung field region can be adjusted based on the maximum value and the minimum value of the pixel values belonging to the lung field region.

A first medical use image A1 normalized by the normalization processing unit 32 is added as input data for training the second learning model 18 via the switch 36A.

On the other hand, the uncertainty value calculation unit 34 calculates a value of uncertainty C1 of the first medical use image a1 input to the first learning model 16 based on the uncertainty c1 estimated by the first learning model 16. The value of uncertainty C1 can be calculated as a statistic converted per unit area of the lung field region by using the predictive variance for each pixel of the lung field region. For example, the proportion of pixels with high uncertainty in the entire lung field region can be used as the value of uncertainty (index) of the first medical use image a1.

The value of uncertainty C1 calculated by the uncertainty value calculation unit 34 is output to the selection unit 36.

The selection unit 36 is a unit that selects the first medical use image a1 suitable for the machine learning (excludes an inappropriate first medical use image a1) from among a large number of the first medical use images a1 constituting the large-scale data set B. The selection unit 36 compares the value of uncertainty C1 with a threshold value Th. In a case in which the value of uncertainty C1 is smaller than the threshold value Th, the selection unit 36 determines that the first medical use image a1 is an image with high reliability (image suitable for creating the second learning model 18 having high reliability of detection of the disease), turns on each of the switches 36A and 36B, adds the normalized first medical use image A1 to the second learning model 18 as the input data, and adds the disease label x corresponding to the first medical use image A1 to the error calculation unit 38 as the correct answer label.

As a result, it is possible to select the first medical use image A1 suitable for learning, which has less noise and blurriness and less obstructive shadow (shadow of a heart pacemaker or the like).

It should be noted that, although the selection unit 36 according to this example selects the first medical use image A1 after the normalization, the selection unit 36 may also select the first medical use image a1 before the normalization. In this case, the first medical use image a1 that is not selected is not subjected to the normalization processing, and the waste of the processing can be eliminated.

The second learning model 18 has been trained by using the first medical use image A1, which is selected by the selection unit 36 and is normalized, among the first medical use image group with disease label constituting the data set B as the input data and using the disease label x added to the input data as the correct answer label.

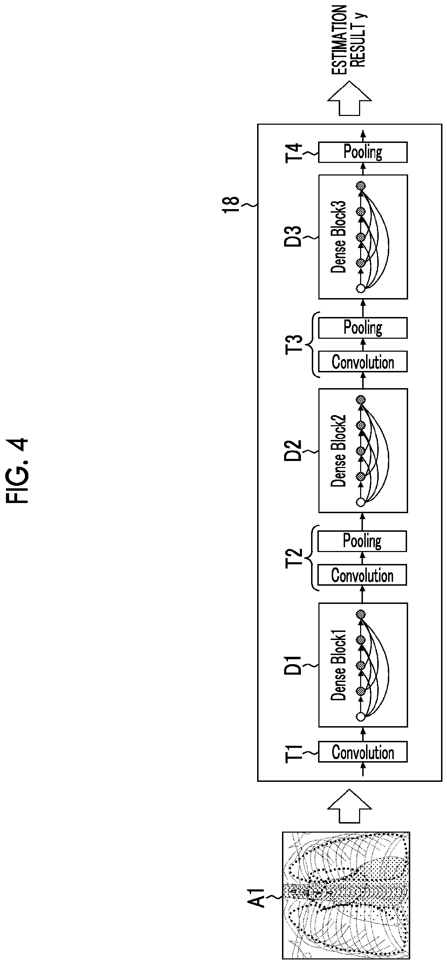

Fig. 4 is a diagram showing an example of the second learning model. The second learning model 18 shown in Fig. 4 is a learning model consisting of the DenseNet as described above.

As shown in Fig. 4, the second learning model 18 has a network structure including a plurality of dense blocks D1 to D3 and a plurality of transition layers T1 to T4 before and behind the dense blocks D1 to D3, and showing high performance in the task of class-classification. In the dense blocks D1 to D3, the reduction of the gradient disappearance is performed by imposing the skip connection on all the layers. A convolutional layer and/or a pooling layer are provided as the transition layers T1 to T4.

The first medical use image A1, which is selected by the selection unit 36 and is normalized, is input to the second learning model 18 as the image for learning, and the second learning model 18 extracts a feature from the first medical use image A1, and outputs an estimation result y indicating the presence or absence of the disease (lung nodule). Since the second learning model 18 is trained to classify the first medical use image A1 into two categories, "with lung nodule" or "without lung nodule", the estimation result y is output as two scores (total of the two scores is 100%) corresponding to "with lung nodule" and "without lung nodule".

Returning to Fig. 2, the estimation result y indicating the presence or absence of the lung nodule, which is estimated by the second learning model 18 from the normalized first medical use image A1, and the disease label x added to the first medical use image a1 before the normalization of the first medical use image A1 are added to the error calculation unit 38, and the error calculation unit 38 calculates an error between the estimation result y and the disease label x. As the method of calculating the error, for example, softmax cross entropy or sigmoid can be considered.

The learning control unit 40 adjusts a coefficient of a filter, an offset value, and a weight of the connection between the before-and-behind layers applied to the convolutional layer in the second learning model 18 by an error back propagation method based on the error calculated by the error calculation unit 38.

The learning control unit 40 repeatedly executes the processing of adjusting the coefficient and the like in the second learning model 18, and performs learning such that a difference between the estimation result y of the second learning model 18 and the disease label x is small.

The learning control unit 40 or the like makes the second learning model 18 that has not been trained to the trained learning model, by advancing training the second learning model 18 with one pair of the first medical use image A1 after the normalization of the first medical use image a1 in which the value of uncertainty C1 is smaller than the threshold value Th and the disease label x in the data set B stored in the memory 14, or in units of mini-batch of around 10 to 100 and performing learning such that the coefficient and the like in the second learning model 18 is optimized.

With the learning device 10-1 according to the first embodiment, it is possible to use the data set for learning (large-scale data set B) having a low annotation cost. Also, since the first medical use image a1 (image in which the value of uncertainty C1 is equal to or larger than the threshold value Th), which is inappropriate for the machine learning, is automatically excluded from among a large number of first medical use images a1 constituting the data set B, and the second learning model 18 that detects the lung nodule is trained by using the first medical use image A1 after the normalization of the first medical use image a1 that is appropriate for the machine learning based on the lung field region, it is possible to create the second learning model 18 with high reliability of the lung nodule detection.

<Second Embodiment of Learning Device>

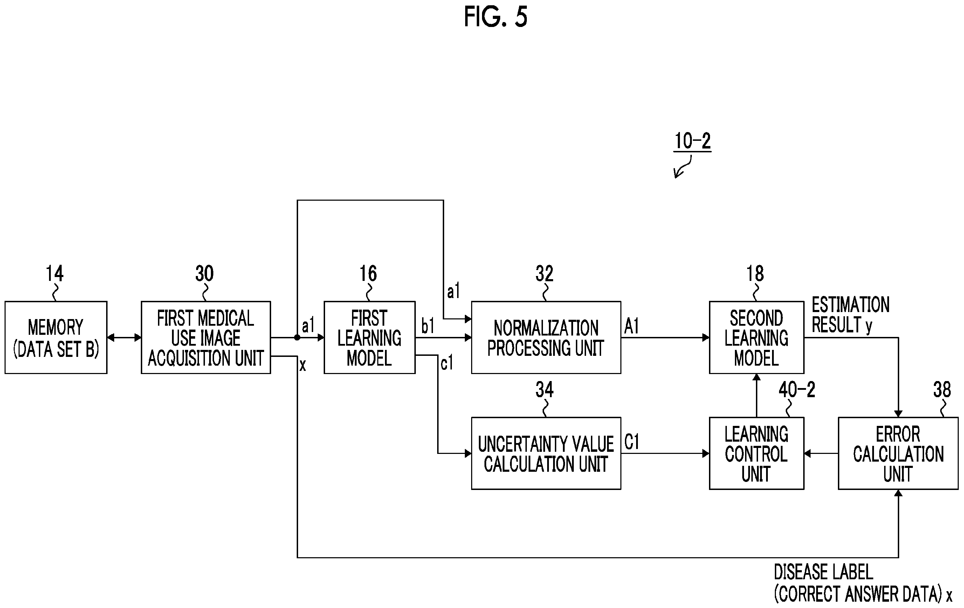

Fig. 5 is a functional block diagram showing a second embodiment of the learning device according to the embodiment of the present invention. It should be noted that, in Fig. 5, the same reference numerals are given to the portions common to the learning device 10-1 according to the first embodiment shown in Fig. 2, and the detailed description thereof will be omitted.

A learning device 10-2 according to the second embodiment shown in Fig. 5 is different from the learning device 10-1 according to the first embodiment in that the selection unit 36 that automatically excludes the first medical use image a1 inappropriate for learning from the data set B, and a learning control unit 40-2 is provided instead of the learning control unit 40.

In the learning control unit 40-2 of the learning device 10-2, the value of uncertainty C1 calculated by the uncertainty value calculation unit 34 is added in addition to the error between the estimation result y calculated by the error calculation unit 38 and the disease label x, and the learning control unit 40-2 weights the error between the estimation result y and the disease label x according to the value of uncertainty C1 and trains the second learning model 18 based on the weighted error.

As the value of uncertainty C1 of the first medical use image a1 is larger, the first medical use image a1 has a more noise and the like and is considered to be more inappropriate as the medical use image for learning. Therefore, in a case in which the first medical use image A1 after the normalization of the first medical use image a1 having a large value of uncertainty C1 is input to the second learning model 18, the learning control unit 40-2 weights the error by multiplying the error calculated corresponding to the first medical use image A1 by a weight (weight in a range of 0 to 1) such that the contribution of the first medical use image A1 to training the second learning model 18. The weighting of the error may be performed continuously or stepwise according to the value of uncertainty C1. Also, in a case in which the learning is performed in units of mini-batch, the weighting may be performed such that the error with respect to the first medical use image a1 in which the value of uncertainty C1 is equal to or larger than the threshold value Th is not used.

With the learning device 10-2 according to the second embodiment, it is possible to use the data set for learning (a large-scale data set B) having a low annotation cost as in the learning device 10-1 according to the first embodiment. In addition, since the first medical use image A1 after the normalization of each of the first medical use images a1 constituting the data set B based on the lung field region is used, the error between the estimation result y and the disease label x is weighted according to the value of uncertainty C1, and the second learning model 18 is trained based on the weighted error, it is possible to create the second learning model 18 with high reliability that reflects the value of uncertainty C1.

It should be noted that, in the first and second embodiments, the first medical use image a1 is normalized based on the lung field region estimated from the first medical use image a1 by the first learning model 16, and the normalized first medical use image A1 is used as the input data of the second learning model 18, but the present invention is not limited to this. Information indicating the organ region (lung field region) estimated by the first learning model 16 may be set as a different channel from the first medical use image a1 and the first medical use image a1 and the information indicating the lung field region may be used as the input data of the second learning model 18. As the information indicating the lung field region, a bounding box surrounding the lung field region, a mask image for distinguishing the lung field region from another region, or the like can be considered.

[Learning Method]

<First Embodiment of Learning Method>

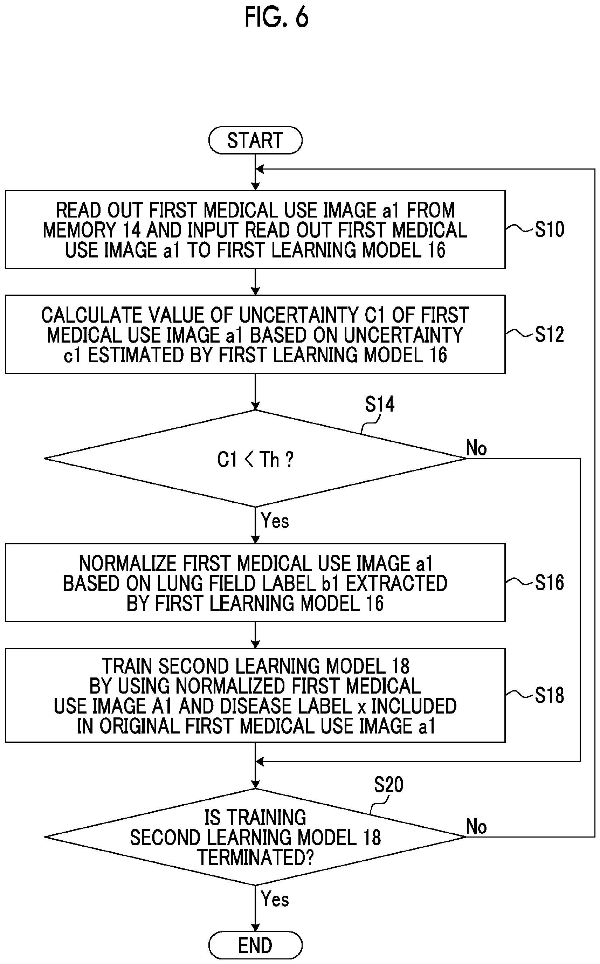

Fig. 6 is a flowchart showing a first embodiment of the learning method according to the embodiment of the present invention.

The processing of each step of the learning method shown in Fig. 6 is processing performed by the learning device 10-1 according to the first embodiment shown in Fig. 2.

In Fig. 6, the first medical use image acquisition unit 30 reads out the first medical use image a1 with the disease label from the memory 14 that stores the data set B for learning, and inputs the read out first medical use image a1 to the first learning model 16 (step S10).

The first learning model 16 simultaneously estimates the lung field label b1 and the uncertainty c1 for the extraction of the lung field region from the input first medical use image a1. The uncertainty value calculation unit 34 calculates the value of uncertainty C1 of the first medical use image a1 input to the first learning model 16 based on the uncertainty c1 estimated by the first learning model 16 (step S12).

The selection unit 36 compares the value of uncertainty C1 with the threshold value Th, and the processing transitions to step S16 in a case in which the value of uncertainty C1 is smaller than the threshold value Th (in a case of "Yes") and transitions (jumps) to step S20 in a case in which the value of uncertainty C1 is equal to or larger than the threshold value Th (in a case of "No") (step S14). As a result, the first medical use image a1 in which the value of uncertainty C1 is smaller than the threshold value Th is adopted as the learning data of the second learning model 18, and the first medical use image a1 in which the value of uncertainty C1 is equal to or larger than the threshold value Th is excluded from the learning data of the second learning model 18.

In step S16, the normalization processing unit 32 normalizes the first medical use image a1 based on the lung field region indicated by the lung field label b1. The processing of normalization can be performed, for example, by cutting out the image from the first medical use image a1 with the bounding box surrounding the lung field region.

Next, the second learning model 18 is trained by using the normalized first medical use image A1 and the disease label x included in the original first medical use image a1 before the normalization (step S18). That is, the error calculation unit 38 calculates the error between the estimation result y estimated by the second learning model 18 from the first medical use image A1 and the disease label x, and the learning control unit 40 trains the second learning model 18 by adjusting the parameters such as the coefficient of the filter in the second learning model 18 by the error back propagation method based on the calculated error.

Subsequently, it is determined whether or not training the second learning model 18 is terminated (step S20). In a case in which it is determined that the learning is not terminated, the processing transitions to step S10 and the pieces of processing of step S10 to step S20 are repeatedly executed, and in a case in which it is determined that the learning is terminated, training the second learning model 18 is terminated. The determination of whether or not training the second learning model 18 is terminated can be performed, for example, by determining whether or not all the first medical use images a1 having a small value of uncertainty C1 in the data set B stored in the memory 14 are used for training the second learning model 18.

<Second Embodiment of Learning Method>

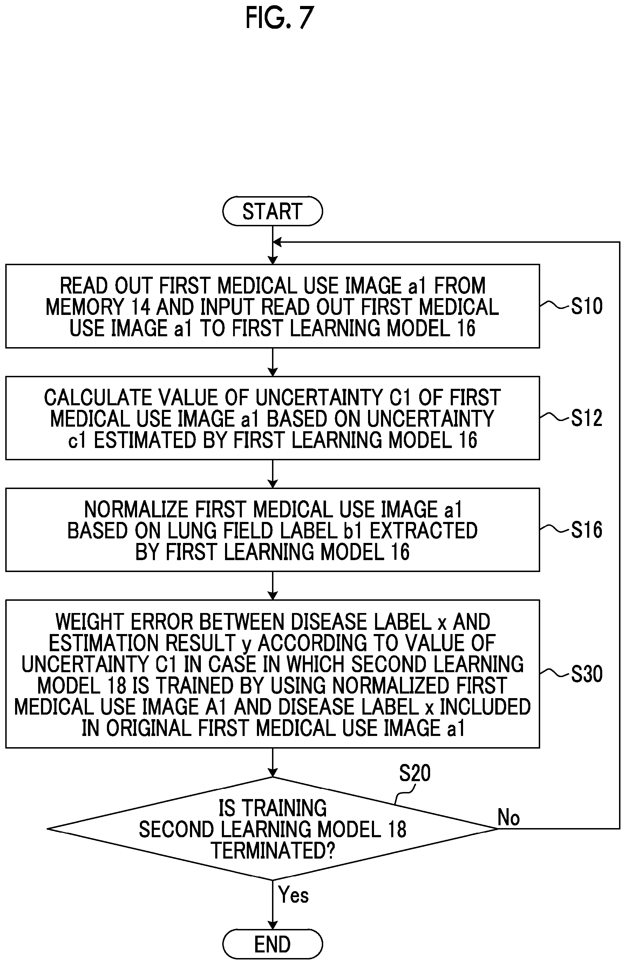

Fig. 7 is a flowchart showing a second embodiment of the learning method according to the embodiment of the present invention.

The processing of each step of the learning method according to the second embodiment shown in Fig. 7 is the processing performed by the learning device 10-2 according to the second embodiment shown in Fig. 5. It should be noted that, in Fig. 7, the same step numbers are given to the portions common to the learning method according to the first embodiment shown in Fig. 6, and the detailed description thereof will be omitted.

The learning method according to the second embodiment shown in Fig. 7 is different in that the processing of step S14 shown in Fig. 6 is not performed and the processing of step S30 is performed instead of step S18 shown in Fig. 6.

The processing of step S30 is processing performed by the learning control unit 40-2 of the learning device 10-2 and processing of training the second learning model 18 by using the normalized first medical use image A1 and the disease label x included in the original first medical use image a1 before the normalization, and is different from the processing of step S18 shown in Fig. 6 in that, in particular, the error between the disease label x and the estimation result y of the second learning model 18 is weighted according to the value of uncertainty C.

For example, in a case in which the normalized first medical use image A1 of the first medical use image a1 having a large value of uncertainty C1 is used for training the second learning model 18, the error is weighted by multiplying the error calculated corresponding to the first medical use image A1 by the weight (weight in a range of 0 to 1). As a result, the contribution of the first medical use image a1 having a large value of uncertainty C1 to training the second learning model 18 is reduced, and the second learning model 18 with high detection accuracy of the disease or the like can be created.

[Medical Use Image Processing Device]

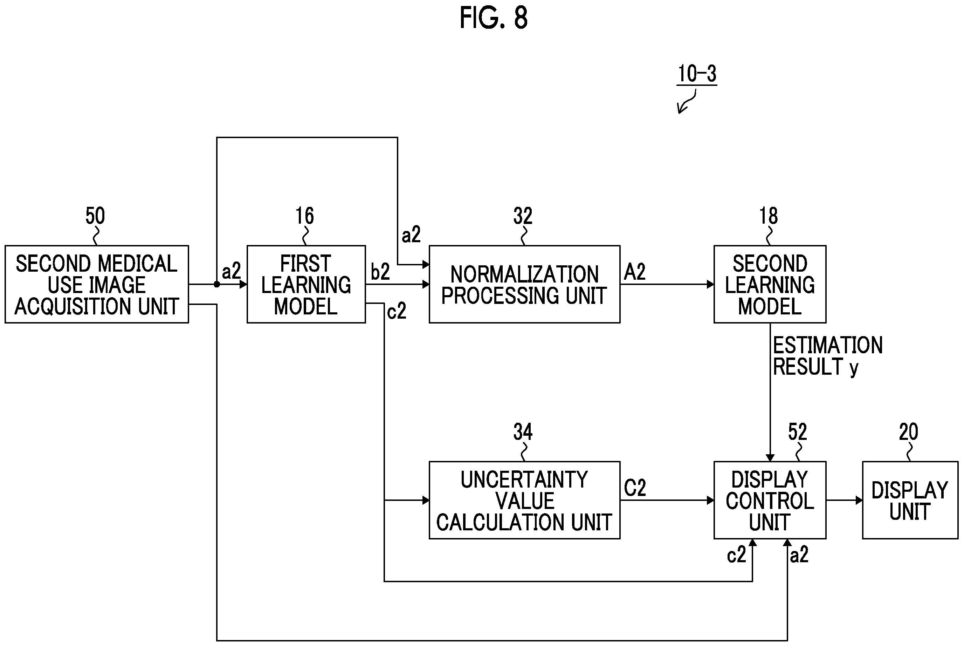

Fig. 8 is a functional block diagram showing an embodiment of the medical use image processing device according to the embodiment of the present invention.

It should be noted that, in Fig. 8, the same reference numerals are given to the portions common to the learning device 10-1 according to the first embodiment shown in Fig. 2, and the detailed description thereof will be omitted.

A medical use image processing device 10-3 shown in Fig. 8 is configured by the processor 12 (second processor), the memory 14, the first learning model 16, the second learning model 18, and the display unit 20 provided in the medical use image processing device 10 of the structure having the hardware configuration shown in Fig. 1, and the processor 12 functions as a second medical use image acquisition unit 50, the normalization processing unit 32, the uncertainty value calculation unit 34, and a display control unit 52.

In addition, the first learning model 16 and the second learning model 18 shown in Fig. 8 are trained learning models, respectively. In particular, the second learning model 18 is the trained learning model that has been trained by the learning device 10-1 according to the first embodiment shown in Fig. 2 or the learning device 10-2 according to the second embodiment shown in Fig. 5.

The medical use image processing device 10-3 according to this example detects the disease (for example, the presence or absence of the lung nodule) from the second medical use image which is the chest X-ray image of a patient, and presents the detection result or the like to the user (doctor) to support the interpretation of the second medical use image by the user.

In Fig. 8, the second medical use image acquisition unit 50 is a unit that acquires a medical use image (second medical use image) a2 of the target for the detection of the disease, the user (doctor) operates the operation unit 24 to acquire any second medical use image a2 that the user intends to interpret via the input/output interface 22, or read out (acquire) the second medical use image a2 from the memory 14 in a case in which the second medical use image a2 is stored in the memory 14.

The second medical use image a2 acquired by the second medical use image acquisition unit 50 is output to each of the first learning model 16, the normalization processing unit 32, and the display control unit 52.

The first learning model 16 simultaneously estimates the extraction of the organ region (lung field region) and uncertainty c2 for the extraction of the lung field region from the input second medical use image a2. A lung field label b2 indicating the lung field region estimated by the first learning model 16 is output to the normalization processing unit 32, and the simultaneously estimated uncertainty c2 is output to each of the uncertainty value calculation unit 34 and the display control unit 52.

The second medical use image a2 is added to another input of the normalization processing unit 32, and the normalization processing unit 32 normalizes the second medical use image a2 based on the lung field label b2 (lung field region indicated by the lung field label b2). The second medical use image A2 normalized by the normalization processing unit 32 is input to the second learning model 18.

The second learning model 18 that has been trained extracts a feature from the second medical use image A2 in a case in which the normalized second medical use image A2 is input, and outputs the estimation result y indicating the presence or absence of the disease (lung nodule) to the display control unit 52.

On the other hand, the uncertainty value calculation unit 34 calculates a value of uncertainty C2 of the second medical use image a2 input to the first learning model 16 based on the uncertainty c2 estimated by the first learning model 16, and outputs the calculated value of uncertainty C2 to the display control unit 52. For the value of uncertainty C2 of the second medical use image a2, for example, the proportion of pixels with high uncertainty c2 in the entire lung field region can be used as the value of uncertainty (index).

The display control unit 52 displays the second medical use image a2 that is the diagnosis target acquired by the second medical use image acquisition unit 50 on the display unit 20, and also displays various information on the display unit 20 based on the uncertainty c2 estimated by the first learning model 16, the value of uncertainty C2 calculated by the uncertainty value calculation unit 34, and the estimation result y estimated by the second learning model 18.

For example, the display control unit 52 can display the estimation result, such as "with suspicion of lung nodule" or "without suspicion of lung nodule", based on the estimation result y estimated by the second learning model 18 on the display unit 20 together with the second medical use image a2 that is the diagnosis target.

In addition, the display control unit 52 can determine whether or not the value of uncertainty C2 of the second medical use image a2 is smaller than the threshold value Th, and notify the user of a warning indicating that the second medical use image a2 is inappropriate as the input data of the second learning model 18 through the display unit 20 in a case in which it is determined that the value of uncertainty C2 of the second medical use image a2 is larger than the threshold value Th.

Further, in a case in which it is determined that the value of uncertainty C2 of the second medical use image a2 is larger than the threshold value Th, the display control unit 52 can display the uncertainty c2 of the lung field region of the second medical use image a2 as well as the second medical use image a2 as a heat map on the display unit 20.

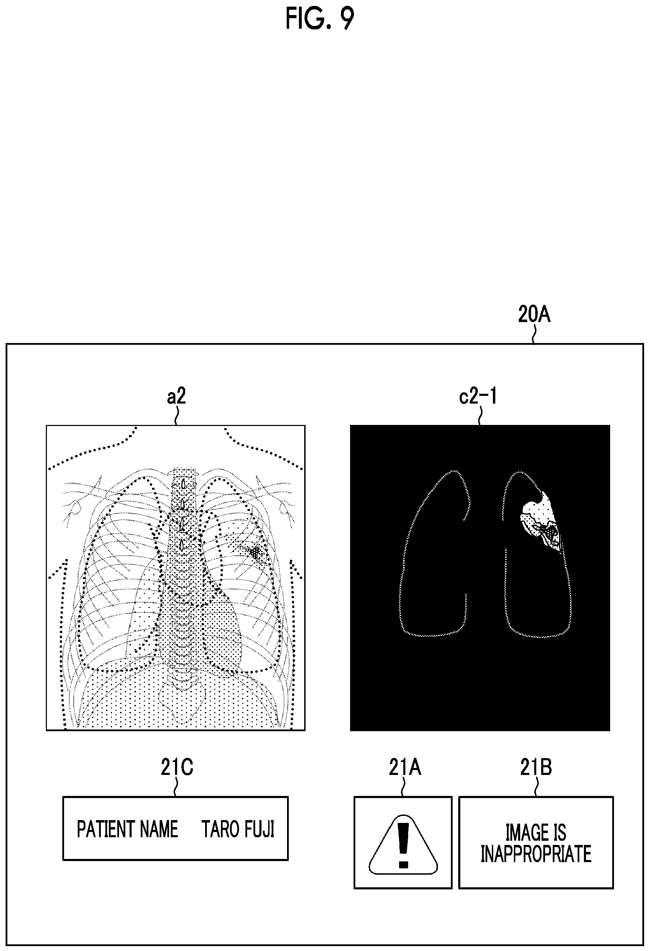

Fig. 9 is a diagram showing a first display example on the display screen of the display unit.

A display screen 20A of the display unit 20 shown in Fig. 9 shows the first display example in a case in which it is determined that the value of uncertainty C2 of the second medical use image a2 is larger than the threshold value Th.

On the display screen 20A of the display unit 20 shown in Fig. 9, a warning icon 21A and text information 21B are displayed together with the second medical use image a2 as the warning indicating that the second medical use image a2 is inappropriate, and a heat map c2-1 corresponding to the uncertainty c2 in the lung field region is further displayed. Also, patient information 21C such as a patient name corresponding to the displayed second medical use image a2 is displayed on the display screen 20A.

The user can recognize that the second medical use image a2 is at least an image inappropriate for the detection of the disease by the second learning model 18 by the warning displayed on the display screen 20A of the display unit 20. In addition, by comparing the second medical use image a2 with the heat map c2-1, it is possible to recognize which pixel or region of the second medical use image a2 has a larger uncertainty c2.

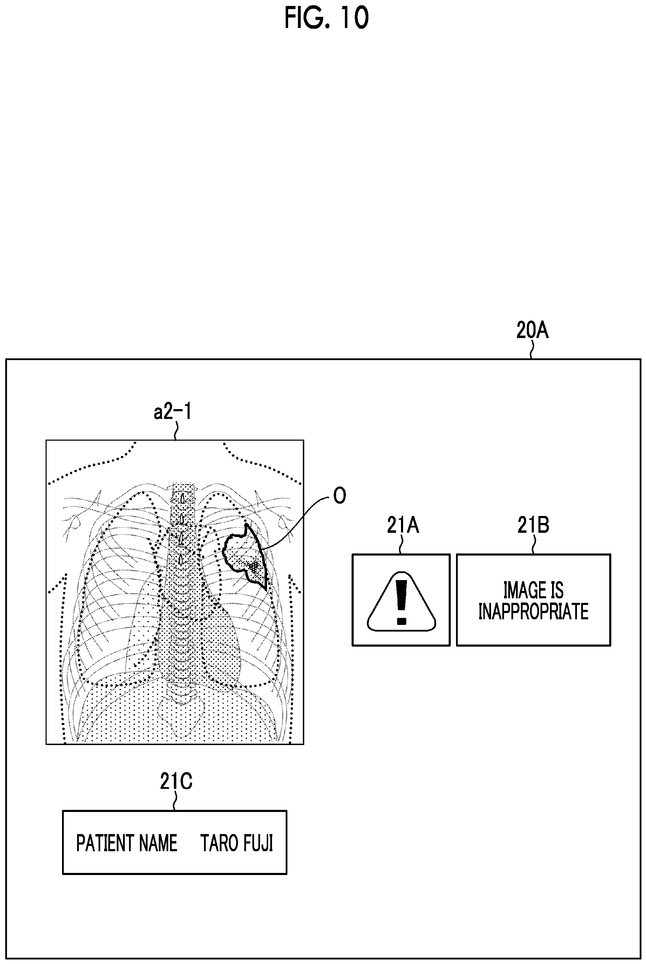

Fig. 10 is a diagram showing a second display example on the display screen of the display unit.

The display screen 20A of the display unit 20 shown in Fig. 10 shows the second display example in a case in which it is determined that the value of uncertainty C2 of the second medical use image a2 is larger than the threshold value Th. It should be noted that, in Fig. 10, the same reference numerals are given to the portions common to the display on the display screen 20A according to the first display example shown in Fig. 9, and the detailed description thereof will be omitted.

The display screen 20A according to the second display example shown in Fig. 10 is different from the display screen 20A according to the first display example shown in Fig. 9 in that the heat map c2-1 shown in Fig. 9 is not displayed, and a second medical use image a2-1 in which a contour O of a region obtained by binarizing the uncertainty c2 of the lung field region of the second medical use image a2 is superimposed on the second medical use image a2 is displayed.

That is, the display control unit 52 compares the uncertainty c2 of the lung field region of the second medical use image a2 with a threshold value set for binarization, extracts the region having the uncertainty c2 larger than the threshold value, and highlights the contour O of the extracted region.

As a result, in a case in which a warning indicating that the second medical use image a2 is inappropriate is displayed, the user can check which region of the lung field region of the second medical use image a2 is inappropriate (which region of the lung field region has a large uncertainty c2).

It should be noted that the display control unit 52 is not limited to the case in which the contour O of the extracted region is highlighted, and may display a circumscribing figure (bounding box) of the extracted region. In addition, since the highlighted contour O or the displayed bounding box may interfere with the interpretation of the second medical use image a2 by the user, it is preferable that the display of the highlighted contour O or the bounding box can be turned on and off by the user operation of the operation unit 24.

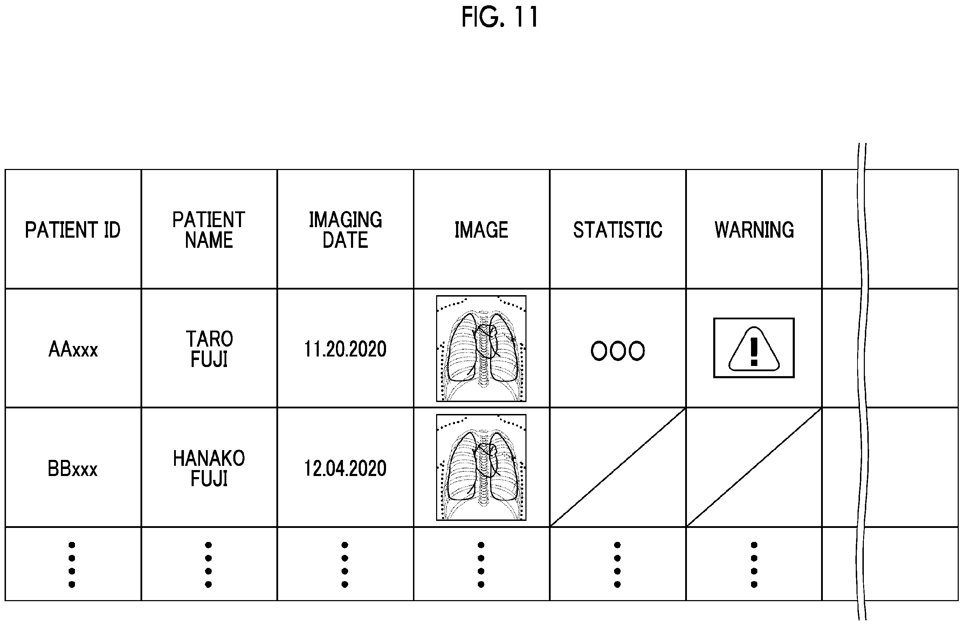

Fig. 11 is a chart showing an example of a patient list.

The second medical use image a2 that is the diagnosis target is stored in the memory 14 shown in Fig. 14 or a memory accessible via the input/output interface 22 in association with the patient information, and it is preferable to create the patient list shown in Fig. 11 and store the created patient list in the memory 14 or the like.

In this case, the display control unit 52 can read out the patient list from the memory 14 or the like in response to the user operation of the operation unit 24 and display the patient list on the display unit 20.

The patient list shown in Fig. 11 is a management table that can manage information in item columns such as "patient name", "imaging date", "image", "statistic", and "warning" in association with user identification (ID) for each patient.

For example, a thumbnail image of the second medical use image a2 is displayed in the column of "image" shown in Fig. 11. It is preferable that the second medical use image a2 corresponding to the thumbnail image is associated with the second medical use image a2, and the display unit 20 displays the second medical use image a2 corresponding to the thumbnail image by clicking the thumbnail image.

In the column of "statistic", the statistic calculated from the second medical use image a2 is displayed in a case in which the value of uncertainty C1 of the second medical use image a2 is larger than the threshold value Th. As the statistic displayed in the column of "statistic", a value converted per unit area of the lung field region using the predictive variance of the uncertainty for each pixel of the lung field region of the second medical use image a2, that is, a scalar value can be used. In addition, in the column of "warning", the warning icon is displayed in a case in which the value of uncertainty C1 of the second medical use image a2 is larger than the threshold value Th.

[Others]

In the present embodiment, the case has been described in which the chest X-ray image is used as the medical use image, but the present invention is not limited to this, and the present invention can also be applied to the medical use images acquired by a computed tomography (CT) apparatus, a magnetic resonance imaging (MRI) apparatus CT, an ultrasound diagnostic apparatus, and the like. In addition, the organ region extracted by the first learning model is not limited to the lung field region and may be two or more different organ regions. The disease detected by the second learning model is not limited to the lung nodule, and one to a plurality of other diseases may be detected.

In addition, in the present embodiment, the case has been described in which the Bayesian U-Net is used as the first learning model, but the present invention is not limited to the Bayesian U-Net, and any learning model may be used as long as the learning model estimates the uncertainty for the extraction of the organ region simultaneously with the extraction of the organ region from the medical use image. Similarly, the case has been described in which the DenseNet is used as the second learning model, but the present invention is not limited to this, and any learning model may be used as long as the learning model can perform the class-classification from the image.

In addition, in the present embodiment, for example, the hardware structure of the processing units that execute various processing, such as the CPU, is the following various processors. The various processors include a central processing unit (CPU), which is a general-purpose processor that executes software (program) and functions as the various processing units, a programmable logic device (PLD), which is a processor of which a circuit configuration can be changed after manufacture, such as a field programmable gate array (FPGA), and a dedicated electric circuit, which is a processor having a circuit configuration that is designed for exclusive use in order to execute specific processing, such as an application specific integrated circuit (ASIC).

One processing unit may be configured by one of these various processors, or may be configured by two or more same type or different types of processors (for example, a plurality of FPGAs or a combination of the CPU and the FPGA). Moreover, a plurality of processing units may be configured by one processor. As a first example the configuration of the plurality of processing units by one processor, there is a form in which one processor is configured by a combination of one or more CPUs and software, and this processor functions as the plurality of processing units, as represented by a computer, such as a client or a server. Second, there is a form in which a processor, which realizes the functions of the entire system including the plurality of processing units with one integrated circuit (IC) chip, is used, as represented by a system on chip (SoC) or the like. In this way, various processing units are configured by one or more of the various processors described above, as the hardware structure.

In addition, the hardware structures of these various processors are, more specifically, an electric circuit (circuitry) in which the circuit elements, such as semiconductor elements, are combined.

Further, the present invention includes the learning program causing the computer to function as the learning device according to the embodiment of the present invention by being installed in the computer, and a non-volatile storage medium in which the learning program is recorded.

Further, the present invention is not limited to the embodiments, and it is needless to say that the modifications can be made without departing from the spirit of the present invention.

Explanation of References

    10, 10-3: medical use image processing device
    10-1, 10-2: learning device
    12: processor
    14: memory
    16: first learning model
    18: second learning model
    20: display unit
    20A: display screen
    21A: warning icon
    21B: text information
    21C: patient information
    22: input/output interface
    24: operation unit
    30: first medical use image acquisition unit
    32: normalization processing unit
    34: uncertainty value calculation unit
    36: selection unit
    36A: switch
    3 6B: switch
    38: error calculation unit
    40, 40-2: learning control unit
    50: second medical use image acquisition unit
    52: display control unit
    A, B: data set
    A1: first medical use image
    A2: second medical use image
    C1, C2: value of uncertainty
    S10 to S20, S30: step

a1: first medical use image
a2, a2-1: second medical use image
b1, b2: lung field label
c1, c2: uncertainty
c2-1: heat map
x: disease label
y: estimation result

**Claims**

1. A learning device comprising:

   a first processor;
   a memory that stores a data set for learning consisting of a plurality of first medical use images each having a disease label;
   a first learning model that has been trained and estimates extraction of an organ region and uncertainty for the extraction of the organ region from the first medical use image in a case in which the first medical use image is input; and
   a second learning model that has not been trained and detects a disease from any second medical use image, wherein the first processor performs

      processing of reading out the first medical use image from the memory and inputting the read out first medical use image to the first learning model,
      processing of normalizing the first medical use image to be input to the second learning model based on the organ region estimated by the first learning model or setting information indicating the organ region with respect to the first medical use image,
      processing of calculating a value of uncertainty of the first medical use image input to the first learning model based on the uncertainty estimated by the first learning model, and
      processing of training the second learning model by using the normalized first medical use image, or the first medical use image and the information indicating the organ region as input data, and the disease label of the first medical use image as a correct answer label, in which the second learning model is trained by reflecting the calculated value of uncertainty of the first medical use image.

2. The learning device according to claim 1,
   wherein, in the processing of training the second learning model, it is determined whether or not the calculated value of uncertainty of the first medical use image is smaller than a threshold value, and the first medical use image for which it is determined that the value of uncertainty is smaller than the threshold value among the first medical use images constituting the data set is used.

3. The learning device according to claim 1,
   wherein, in the processing of training the second learning model, an error between an estimation result of the second learning model and the disease label is weighted according to the calculated value of uncertainty of the first medical use image, and the second learning model is trained based on the weighted error.

4. The learning device according to any one of claims 1 to 3,
   wherein the processing of normalizing the first medical use image includes processing of cutting out the estimated organ region from the first medical use image.

5. The learning device according to any one of claims 1 to 4,
   wherein, in the processing of normalizing the first medical use image, a pixel value of the first medical use image is normalized by a statistic of a pixel value belonging to the organ region estimated from the first medical use image.

6. The learning device according to any one of claims 1 to 5,
   wherein the first learning model is a learning model consisting of a Bayesian neural network.

7. The learning device according to any one of claims 1 to 6,
   wherein the second learning model is a learning model consisting of densely connected convolutional networks (DenseNet).

8. The learning device according to any one of claims 1 to 7,
   wherein the first medical use image and the second medical use image are chest X-ray images, respectively.

9. The learning device according to any one of claims 1 to 8,
   wherein the disease is a lung nodule.

10. A medical use image processing device comprising:

    the learning device according to any one of claims 1 to 9; and
    a second processor,
    wherein the second processor performs

    processing of inputting the second medical use image to the first learning model, and normalizing the second medical use image based on the organ region estimated by the first learning model or setting the information indicating the organ region with respect to the second medical use image, and
    processing of inputting the normalized second medical use image, or the second medical use image and the information indicating the organ region to the second learning model that has been trained, and detecting the disease from the second medical use image based on an estimation result of the second learning model.

11. The medical use image processing device according to claim 10,
    wherein the second processor performs

    processing of inputting the second medical use image to the first learning model,
    processing of calculating a value of uncertainty of the second medical use image input to the first learning model based on the uncertainty estimated by the first learning model, and determining whether or not the value of uncertainty is smaller than a threshold value, and
    processing of notifying a user of a warning in a case in which it is determined that the value of uncertainty of the second medical use image is larger than the threshold value.

12. The medical use image processing device according to claim 11,

    wherein the value of uncertainty of the second medical use image is calculated as a statistic converted per unit area of the organ region based on the uncertainty of the second medical use image input to the first learning model, and
    in the processing of notifying the user of the warning, an icon indicating the statistic or the warning is displayed.

13. The medical use image processing device according to claim 11,
    wherein, in the processing of notifying the user of the warning, uncertainty of the organ region of the second medical use image as well as the second medical use image is displayed as a heat map.

14. The medical use image processing device according to claim 11,
    wherein, in the processing of notifying the user of the warning, a contour or a circumscribing figure of a region obtained by binarizing uncertainty of the organ region of the second medical use image as well as the second medical use image is displayed.

15. A learning method in which a first processor trains a second learning model that has not been trained and detects a disease from any second medical use image, by using a data set for learning that is stored in a memory and consists of a plurality of first medical use images each having a disease label, and a first learning model that has been trained and estimates extraction of an organ region and uncertainty for the extraction of the organ region from the first medical use image in a case in which the first medical use image is input, the learning method comprising:

    a step of reading out the first medical use image from the memory and inputting the read out first medical use image to the first learning model;
    a step of normalizing the first medical use image to be input to the second learning model based on the organ region estimated by the first learning model or setting information indicating the organ region with respect to the first medical use image;

a step of calculating a value of uncertainty of the first medical use image input to the first learning model based on the uncertainty estimated by the first learning model; and

a step of training the second learning model by using the normalized first medical use image, or the first medical use image and the information indicating the organ region as input data, and the disease label of the first medical use image as a correct answer label, in which the second learning model is trained by reflecting the calculated value of uncertainty of the first medical use image.

16. The learning method according to claim 15,

wherein, in the step of training the second learning model, it is determined whether or not the calculated value of uncertainty of the first medical use image is smaller than a threshold value, and the first medical use image for which it is determined that the value of uncertainty is smaller than the threshold value among the first medical use images constituting the data set is used.

17. The learning method according to claim 15,

wherein, in the step of training the second learning model, an error between an estimation result of the second learning model and the disease label is weighted according to the calculated value of uncertainty of the first medical use image, and the second learning model is trained based on the weighted error.

18. A learning program causing the first processor to execute processing of each step in the learning method according to any one of claims 15 to 17.

19. A non-transitory computer-readable recording medium in which the program according to claim 18 is recorded.

# FIG. 1

FIG. 2

14 MEMORY (DATA SET B)

30 FIRST MEDICAL USE IMAGE ACQUISITION UNIT

16 FIRST LEARNING MODEL

32 NORMALIZATION PROCESSING UNIT

34 UNCERTAINTY VALUE CALCULATION UNIT

36 SELECTION UNIT

36A

36B

18 SECOND LEARNING MODEL

40 LEARNING CONTROL UNIT

38 ERROR CALCULATION UNIT

ESTIMATION RESULT y

DISEASE LABEL (CORRECT ANSWER DATA) x

10-1

a1  b1  c1  A1  C1  x

# FIG. 3

a1: FIRST MEDICAL USE IMAGE

c1: UNCERTAINTY

16

b1: LUNG FIELD LABEL

EP 4 270 305 A1

# FIG. 4

ESTIMATION RESULT y

FIG. 5

$\underline{10\text{-}2}$

**14** MEMORY (DATA SET B)

**30** FIRST MEDICAL USE IMAGE ACQUISITION UNIT

**16** FIRST LEARNING MODEL

**32** NORMALIZATION PROCESSING UNIT

**18** SECOND LEARNING MODEL

ESTIMATION RESULT y

**34** UNCERTAINTY VALUE CALCULATION UNIT

**40-2** LEARNING CONTROL UNIT

**38** ERROR CALCULATION UNIT

DISEASE LABEL (CORRECT ANSWER DATA) x

EP 4 270 305 A1

# FIG. 6

START

READ OUT FIRST MEDICAL USE IMAGE a1 FROM
MEMORY 14 AND INPUT READ OUT FIRST MEDICAL
USE IMAGE a1 TO FIRST LEARNING MODEL 16    ~S10

CALCULATE VALUE OF UNCERTAINTY C1 OF FIRST
MEDICAL USE IMAGE a1 BASED ON UNCERTAINTY
c1 ESTIMATED BY FIRST LEARNING MODEL 16    ~S12

S14

$C1 < Th$ ?    No

Yes

NORMALIZE FIRST MEDICAL USE IMAGE a1
BASED ON LUNG FIELD LABEL b1 EXTRACTED
BY FIRST LEARNING MODEL 16    ~S16

TRAIN SECOND LEARNING MODEL 18
BY USING NORMALIZED FIRST MEDICAL
USE IMAGE A1 AND DISEASE LABEL x INCLUDED
IN ORIGINAL FIRST MEDICAL USE IMAGE a1    ~S18

S20

IS TRAINING
SECOND LEARNING MODEL 18
TERMINATED?    No

Yes

END

# FIG. 7

```
                    ( START )
                        │
                        ▼
┌──────────────────────────────────────────────┐
│   READ OUT FIRST MEDICAL USE IMAGE a1 FROM    │
│  MEMORY 14 AND INPUT READ OUT FIRST MEDICAL   │ ～ S10
│    USE IMAGE a1 TO FIRST LEARNING MODEL 16    │
└──────────────────────────────────────────────┘
                        │
                        ▼
┌──────────────────────────────────────────────┐
│   CALCULATE VALUE OF UNCERTAINTY C1 OF FIRST  │
│  MEDICAL USE IMAGE a1 BASED ON UNCERTAINTY    │ ～ S12
│    c1 ESTIMATED BY FIRST LEARNING MODEL 16    │
└──────────────────────────────────────────────┘
                        │
                        ▼
┌──────────────────────────────────────────────┐
│      NORMALIZE FIRST MEDICAL USE IMAGE a1     │
│   BASED ON LUNG FIELD LABEL b1 EXTRACTED      │ ～ S16
│        BY FIRST LEARNING MODEL 16             │
└──────────────────────────────────────────────┘
                        │
                        ▼
┌──────────────────────────────────────────────┐
│   WEIGHT ERROR BETWEEN DISEASE LABEL x AND    │
│  ESTIMATION RESULT y ACCORDING TO VALUE OF    │
│ UNCERTAINTY C1 IN CASE IN WHICH SECOND LEARNING│ ～ S30
│    MODEL 18 IS TRAINED BY USING NORMALIZED FIRST│
│ MEDICAL USE IMAGE A1 AND DISEASE LABEL x INCLUDED│
│     IN ORIGINAL FIRST MEDICAL USE IMAGE a1    │
└──────────────────────────────────────────────┘
                        │
                        ▼               S20
                   ╱─────────────╲
                  ╱  IS TRAINING   ╲      No
                 ╱ SECOND LEARNING   ╲ ─────────►
                 ╲   MODEL 18       ╱
                  ╲  TERMINATED?   ╱
                   ╲─────────────╱
                        │ Yes
                        ▼
                     ( END )
```

23

# FIG. 8

EP 4 270 305 A1

# FIG. 9

20A

a2

c2-1

21C

PATIENT NAME    TARO FUJI

21A

21B

IMAGE IS
INAPPROPRIATE

# FIG. 10

FIG. 11

| PATIENT ID | PATIENT NAME | IMAGING DATE | IMAGE | STATISTIC | WARNING | | |
|---|---|---|---|---|---|---|---|
| AAxxx | TARO FUJI | 11.20.2020 | | OOO | ⚠ | | |
| BBxxx | HANAKO FUJI | 12.04.2020 | | | | | |
| ⋮ | ⋮ | ⋮ | ⋮ | ⋮ | ⋮ | | |

EP 4 270 305 A1

# INTERNATIONAL SEARCH REPORT

| International application No. |
|---|
| **PCT/JP2021/045790** |

**A. CLASSIFICATION OF SUBJECT MATTER**

*G06T 7/00*(2017.01)i; *A61B 6/00*(2006.01)i; *G06N 20/00*(2019.01)i
FI:   A61B6/00 360Z; G06T7/00 350C; G06T7/00 614; G06N20/00 130

According to International Patent Classification (IPC) or to both national classification and IPC

**B. FIELDS SEARCHED**

Minimum documentation searched (classification system followed by classification symbols)

A61B6/00-6/14; G06T7/00-7/90

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | JP 2020-86519 A (CANON MEDICAL SYSTEMS CORP.) 04 June 2020 (2020-06-04) paragraphs [0020], [0036], [0048], [0064] | 1-19 |
| Y | JP 2020-503603 A (HEARTFLOW INC.) 30 January 2020 (2020-01-30) paragraphs [0014], [0037], [0038], [0040] | 1-19 |
| A | JP 2019-149093 A (FUJIFILM CORP.) 05 September 2019 (2019-09-05) paragraphs [0034]-[0044] | 1-19 |
| A | US 2019/0122073 A1 (THE CHARLES STARK DRAPER LABORATORY, INC.) 25 April 2019 (2019-04-25) paragraph [0036] | 1-19 |

☐ Further documents are listed in the continuation of Box C.  ☑ See patent family annex.

| \* | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **11 January 2022** | **08 March 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

### INTERNATIONAL SEARCH REPORT
**Information on patent family members**

International application No.

**PCT/JP2021/045790**

| Patent document cited in search report | | | Publication date (day/month/year) | Patent family member(s) | | | Publication date (day/month/year) |
|---|---|---|---|---|---|---|---|
| JP | 2020-86519 | A | 04 June 2020 | US 2020/0160981 A1 paragraphs [0035], [0052], [0064], [0081] | | | |
| JP | 2020-503603 | A | 30 January 2020 | US 2018/0182101 A1 paragraphs [0017], [0040], [0041], [0043] WO 2018/119358 A1 EP 3559907 A1 | | | |
| JP | 2019-149093 | A | 05 September 2019 | (Family: none) | | | |
| US | 2019/0122073 | A1 | 25 April 2019 | WO 2019/084028 A1 | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2020091862 A

### Non-patent literature cited in the description

- When does bone suppression and lung field segmentation improve chest x-ray disease classification?. **BALTRUSCHAT et al.** ISBI. 2019
- **GHESU et al.** Quantifying and leveraging classification uncertainty for chest radiograph assessment. *MICCAI,* 2019
- Quantifying and leveraging classification uncertainty for chest radiograph assessment. **GHESU et al.** MICCAI. 2019
- **BALTRUSCHAT et al.** When does bone suppression and lung field segmentation improve chest x-ray disease classification?. *ISBI,* 2019
- **GAL, YARIN ; ZOUBIN GHAHRAMANI.** Dropout as a Bayesian approximation: Representing model uncertainty in deep learning. *ICML,* 2016
- **HIASA ; YUTA et al.** Automated muscle segmentation from clinical CT using Bayesian U-net for personalized musculoskeletal Modeling. *IEEE TMI,* 2019